# EUROPEAN PATENT SPECIFICATION

(11) **EP 2 106 780 B1**
(45) Date of publication and mention of the grant of the patent: **09.03.2016**
(21) Application number: 08425217.0
(22) Date of filing: 03.04.2008
(51) Int. Cl.: A61H 9/00

(54) **Method for treating the scalp to slow and prevent hair loss and kit for performing the method**
Verfahren zur Behandlung der Kopfhaut zur Verlangsamung oder Vorbeugung von Haarausfall und Kit zur Durchführung des Verfahrens
Procédé de traitement du cuir chevelu pour ralentir ou empêcher la chute des cheveux et kit pour effectuer le procédé

(43) Date of publication of application: 07.10.2009
(73) Proprietor: Pomar, Rodolfo, 40067 Pianoro, Frazione Rastignano (BO) (IT)
(72) Inventor: Pomar, Rodolfo, 40067 Pianoro, Frazione Rastignano (BO) (IT)
(74) Representative: Manzella & Associati

(56) References cited:
- EP-A- 1 728 534
- WO-A-2007/073106
- DE-A1- 3 203 922
- DE-C- 848 234
- GB-A- 2 126 900
- US-A- 5 454 778
- US-A1- 2002 188 334
- US-A1- 2004 077 977
- US-A1- 2004 153 131
- US-A1- 2006 161 226
- US-A1- 2007 225 621

## Description

The present invention relates to a method for treating the scalp to slow and prevent hair loss and to a kit for performing the method.

It is known that by subjecting the skin of a person to appropriate cycles of lighting with light beams of preset wavelengths it is possible to treat certain blemishes and contrast certain problems of the skin.

The design choice that entails the greatest advantages in skin treatment provides for the use of small units (provided with a light source) which can be directed toward a small part of the skin and arranged at a short distance from it.

Among these, the embodiment disclosed in EP1535582 offers the extremely advantageous possibility to substantially replace the source (or a filter interposed between the source and the skin), using for each type of skin the most suitable light source in combination with an appropriate treatment cycle.

The spread of light-emitting diodes and the corresponding extension of their possible fields of application has recently led some manufacturers to study skin treatment devices which use diodes as light sources.

Among the advantages provided by diodes with respect to other sources, besides the fact that their first faults linked to aging occur after far longer times than other light sources, the extreme precision of the wavelength of the emitted beam has to be included: this entails first of all the possibility to use no filters to eliminate dangerous frequencies and secondly a great specificity of each treatment.

Among these, the embodiment disclosed in EP 1728534 offers the extremely advantageous possibility to use LEDs which have different characteristics for the aesthetic treatment of the skin.

Androgenetic alopecia, or common baldness, is a thinning of the frontal-occipital portion of head hair, due to miniaturization of the shaft.

Androgenetic alopecia is linked to the transformation of testosterone into dihydrotestosterone, which is particularly concentrated in the frontal regions with respect to the occipital ones: this transformation leads to a weakening of the shaft, with consequent hair loss. In women, the course can be accelerated by particular endocrine conditions (menopause, ovarian polycystosis, virilizing tumors) or by androgen therapy. The resulting shaft is thinner and shorter than normal. At a later age, atrophy of the scalp is also added, and loss of individuality of the papillary cycles which is characteristic of normal adult scalp, and therefore of their synchronization, also occurs.

These phenomena have been contrasted by means of pharmaceutical and mechanical treatments (such as massages and the like). All these attempts have never led to assured elimination of the problem and indeed often no positive effect can be documented for these treatments.

DE 848234 discloses an apparatus comprising a helmet provided with devices for the treatment of the scalp of a user by negative or positive pressure, with ultraviolet light by a mercury lamp or by electrophoresis.

The aim of the present invention is to obviate the cited drawbacks and meet the mentioned requirements, by providing a method for treating the scalp to slow and prevent hair loss which is particularly effective and suitable for application to any individual.

Within this aim, an object of the present invention is to provide a kit for performing the method which is adapted to be controlled by existing apparatuses.

Another object of the present invention is to provide a kit for performing the method which is adapted to perform a targeted and simultaneous action on all the scalp.

Another object of the present invention is to achieve said aim with a structure which is simple, relatively easy to provide in practice, safe in use, effective in operation, and of relatively low costs.

This aim and these and other objects which will become better apparent hereinafter are achieved by the present cosmetic method for treating the scalp to slow and prevent hair loss, comprising the steps set forth in claim 1.

The invention further regards a kit for performing the cosmetic treatment of claim 1, that has the features set forth in claim 4.

Further characteristics and advantages of the invention will become better apparent and evident from the following detailed description of a preferred but not exclusive embodiment of a method for treating the scalp to slow and prevent hair loss and of a kit for performing the method, illustrated by way of non-limiting example in the accompanying drawings, wherein:

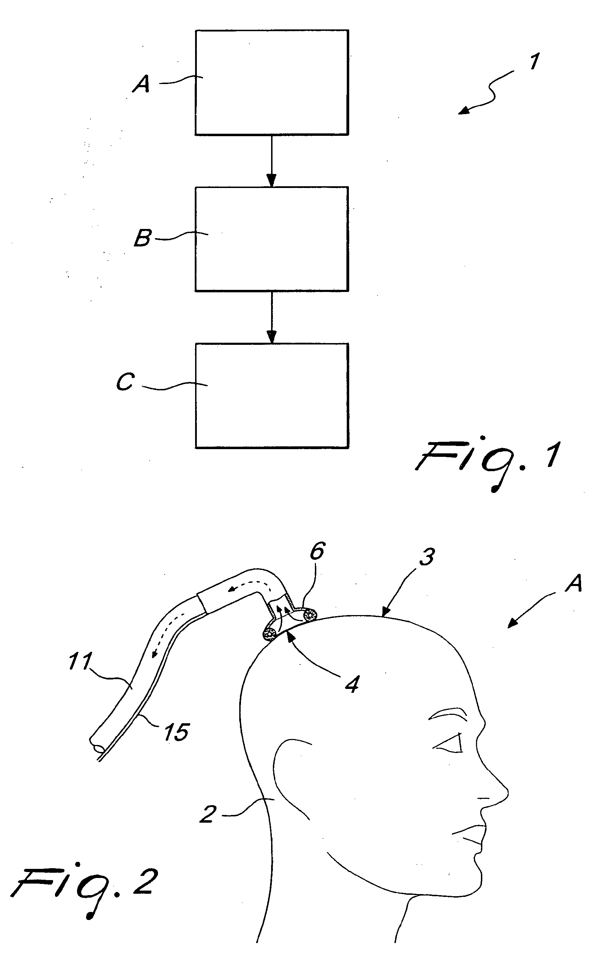

Figure 1 is a block diagram of the steps of a method according to the invention;
Figure 2 is a schematic side view of a step of the method according to the invention;

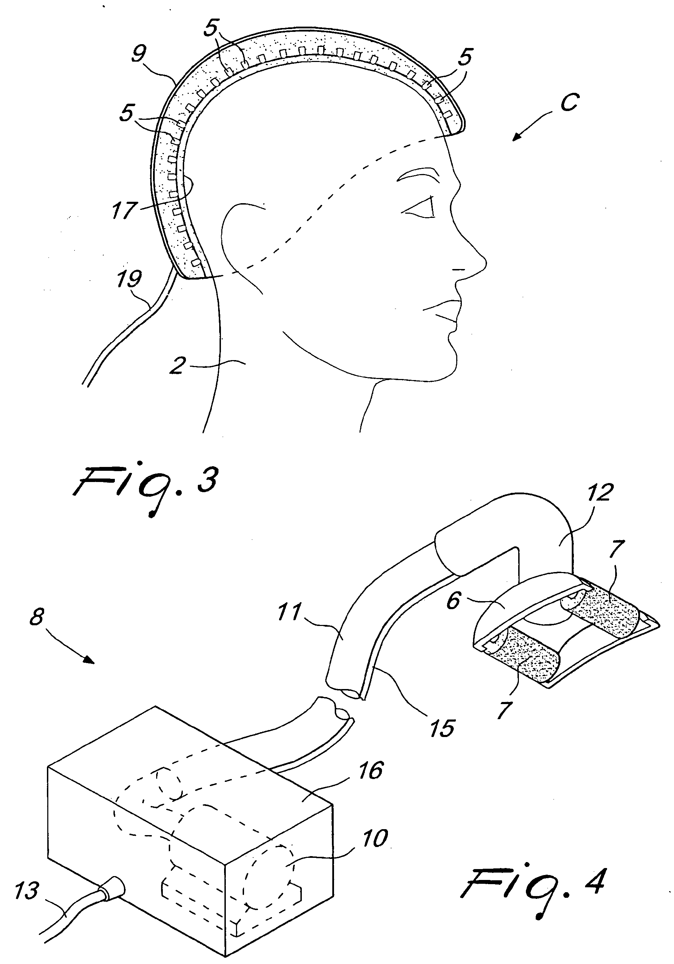

Figure 3 is a schematic side view of a step of the method according to the invention;
Figure 4 is a perspective view of a component of the kit according to the invention;

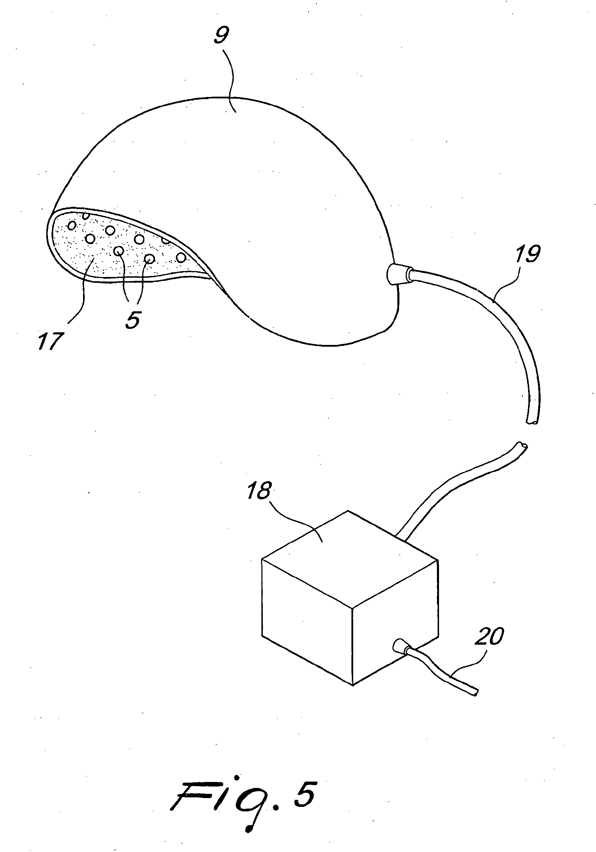

Figure 5 is a perspective view of another component of the kit according to the invention.

With reference to the figures, the reference numeral 1 generally designates a method for treating the scalp to slow and prevent hair loss.

As regards hair thinning problems, and particularly with reference to the so-called androgenetic alopecia, it would be advisable to resort to non-pharmacological therapies to stop hair loss and stimulate hair regrowth. This choice would provide assurance to patients 2 that the treatments they undergo have the least repercussions, unwanted effects which are typical of drugs, on them.

During this process of gradual hair loss, hair begins to thin and to become increasingly fine, producing an effect which is similar to faint down.

The method 1 according to the invention attempts to mutually combine a plurality of medical and aesthetic techniques adopted mostly in other fields, in order to obtain therefrom a synergistic effect which ensures blocking and possible regression of thinning.

One of the technologies that is adopted is known as photobiostimulation and produces an increase in peripheral vascularization and cell metabolism of a scalp 3. Head hair grows stronger, with a larger diameter, more resistant to traction and with a healthier appearance; after biostimulation of the tissues, the skin also appears improved.

In combination with this photobiostimulation, an attempt is made to ensure a great increase in surface microcirculation.

This method 1 is therefore effective on two fronts: in controlling hair loss and in stimulating the follicles from which new healthier and stronger hair derives.

The method 1 for treating the scalp 3 to slow and prevent hair loss therefore consists of a sequence of operating steps.

During the first step A it is necessary to perform mechanical massage; this massage must comprise a suction, known as skin suction, whose intensity is variable as a function of the type of treatment that is intended to be performed, of at least one portion 4 of the surface of the scalp 3: in particular, it is possible to locate this massage with skin suction in a particular region, for example the one in which thinning is more conspicuous, or perform it on the entire scalp 3, since such massage, in addition to being pleasant, provides benefit also to areas not affected by alopecia.

During the second step B, it is convenient to arrange on the at least one portion 4 of the surface of the scalp 3 a mixture which comprises, in proportions which can vary from 0.01% to 30%, a chemical compound having the formula C₉H₁₅N₅O, known by the trade name Minoxidil®.

It should be noted appropriately that the mixture might also be distributed before the first step A, or that the distribution of the mixture (step B) might be followed by a massage with suction (step A) in order to facilitate absorption of the mixture by the scalp 3.

The chemical compound known by the trade name Minoxidil® was discovered in 1980 and was tested initially to contrast hypertension, since when used orally it has a powerful vasodilatory activity. A particular side effect, characterized by body hair and head hair growth, hirsutism, was observed. Testing was thus begun with the purpose of contrasting androgenetic alopecia.

Indeed, Minoxidil® is currently still widespread and used, also in combination with other topical products, to contrast androgenetic alopecia, especially in males: it reduces or stops hair loss and in some cases facilitates regrowth.

Finally, the method provides for the third step C, during which it is necessary to subject the at least one portion 4 of surface of the scalp 3 to the luminous radiation emitted by light-emitting diodes 5, normally known as LEDs.

In particular, the mechanical massage with suction of the skin performed during the first step A consists in arranging on the portion 4 of surface to be treated of the scalp 3 a suction inlet 6, which is adapted to produce a partial vacuum on the skin that faces the inlet 6 and is delimited thereby.

The suction inlet 6, according to an embodiment of particular interest in practice and in application, can comprise massaging means 7 which are adapted to subject to a mechanical treatment the portion 4 of surface to be treated of the scalp 3 which faces and lies proximate to the inlet 6. It is up to the operator to move the suction inlet 6 along the surface of the scalp 3 so as to treat correctly all the areas that require this type of massage intended to boost surface circulation and microcirculation.

In this regard, it should be noted that the connective tissue supports all the epidermal structures and is the seat of blood and lymphatic microcirculation: indeed, it is the organ that carries and provides functional substances to the scalp 3.

By way of the suction inlet 6 and its massaging means 7, it is possible to perform a sustained mechanical massage of the connective tissue of the entire scalp 3. The method is appropriately managed by means of a computer designed to provide the correct combination of the three basic functions of the massage: strength, frequency and cycle.

In this manner, the opening of the vascular channels (blood and lymphatic channels) is facilitated, vascularization of the region is improved and therefore toxins are eliminated more swiftly, achieving a strong stimulation of elastic fibers.

The massage performed with the suction inlet 6 and its means 7 facilitates and stimulates good drainage of interstitial liquids, increases oxygenation of the scalp 3 and therefore restores correct metabolism.

The standard application parameters can be changed by the operator for optimum comfort of the patient 2 during treatment and to allow individual adaptation of the region to be treated, depending on the greater or smaller presence of hair. The massage of step A, performed with the suction inlet 6, further ensures the reproducibility of the treatment, since it is not linked to the manual skill of the operator.

It should be noted that the step B in which the mixture is distributed can provide for a localized distribution, only in the areas more intensely affected by alopecia, or a diffuse distribution, over the entire surface of the scalp 3, depending on the requirements of embodiment and application.

The mixture is a customized solution which contains, in addition to other active treatment components, the active ingredient Minoxidil® at 2%, or at different percentages for performing particular treatments.

The preparation is capable of stopping and sometimes reversing the progressive miniaturization of hair. The action can be performed only in the presence of a germinal center of the hair and therefore hair growth can never occur in regions other than the scalp 3. The mixture acts by stimulating the hair growth phase (anagen) and the stasis phase (ketogen). Its action is enhanced by synergy with the other two steps (step A and step C) of the method 1.

In summary, absorption of the preparation by the scalp 3 is optimized, allowing its most complete effectiveness. In this manner, the method 1 ensures absorption of the mixture and a therapeutic activity thereof which are distinctly surprising and superior to any other known type of treatment.

In particular, it is important to point out that the exposure to luminous radiation (typical of step C) emitted by the light-emitting diodes 5, LEDs, provides for the use of radiation which provides so-called biostimulation of photoreceptors and of the deeper layers of the skin and of the subcutaneous tissue for better oxygenation and vascularization.

This result is achieved by means of biostimulating radiation which comprises at least one among the following light beams:
- a red light beam to stimulate collagen production,
- a blue light beam to contrast bacterial actions,
- a yellow light beam to stimulate the lymphatic system and the nervous system.

The kit for performing and applying the method 1 comprises a suction apparatus 8 and a photostimulation helmet 9.

The suction apparatus 8 is provided with the at least one end inlet 6 which is adapted to be placed adjacent to the portion 4 of the surface to be treated of the scalp 3.

Suction produces massage of the portion 4 and conveyance of blood in the region: the combination of these two actions ensures an increase in surface circulation and microcirculation.

Further, the suction apparatus 8 comprises at least one suction pump 10, at least one duct 11 which is connected to at least one handpiece 12 which is provided with the at least one suction inlet 6.

The handpiece 12 further comprises the suitable movable elements 7, which face outward proximate to the at least one suction inlet 6, for a mechanical massage action to the portion 4 of the surface to be treated.

The massaging members 7 are connected to a respective power supply circuit 13 by means of a suitable cable 15.

The massaging members 7 may be of any type and may have any shape: this depends on the dimensions of the inlet 6 with which they are associated and on the intensity of the massage to be performed.

The electrical components for management and control, the pump 10 and the control of the apparatus 8 are contained within a suitable unit 16.

The helmet 9 is provided, on its internal surface 17, with a plurality of light-emitting diodes 5, LEDs.

The scalp 3 of the patient 2 who wears the helmet 9 is subjected to luminous biostimulation.

The light-emitting diodes 7, LEDs, provided within the helmet 9 are a plurality, distributed on multiple rows, with the purpose of affecting simultaneously with a light stream the entire scalp 3 with the assurance that the sources (diodes 5) are all simultaneously arranged at the correct distance from the scalp 3, thus offering the best possible efficiency of the treatment.

As mentioned, the diodes 5 are all arranged at the same distance from the portion 4 of the surface to be treated; this distance is calculated as a function of the characteristics of the intensity and frequency of the beam emitted by the particular type of diode 5.

Each helmet 9 can comprise a plurality of diodes 5 of a same type or of different types, with the purpose of combining the light beams emitted thereby, taking advantage of the various beneficial effects thereof.

For example, it is possible to adopt diodes 5 which are adapted to emit a red light beam, which is particularly suitable to stimulate the production of collagen.

As an alternative, it is possible to use diodes 5 which are adapted to emit a blue light beam with the purpose of contrasting bacterial actions.

Finally, it is possible to adopt diodes 5 which are adapted to emit a yellow light beam to stimulate the lymphatic system and the nervous system.

The helmet 9 is connected to a control assembly 18 by means of a suitable cable 19. The control assembly 18 is intended to control and manage the operation of the helmet 9: it is also possible to use the same unit 16 of the apparatus 8 also to manage the helmet 9. In this case, the cable 19 is connected directly to the unit 16.

In any case, the unit 18 is powered by means of a suitable cable 20.

Use of LEDs in the medical-aesthetic field became possible only at the end of the 1990s thanks to the birth of new-generation high-pressure LEDs.

Starting from this technological innovation, research was able to develop LEDs with different wavelengths, with a photon density useful for the extended treatment of an area of human tissue.

The action of various wavelengths of the light beams emitted by LEDs allows to obtain an important biological action: biostimulation of the photoreceptors and of the deeper layers of the skin and of the subcutaneous layer, with consequent stimulation of a chain of biophysical reactions which leads to faster and more efficient cell response. A greater supply of oxygen and better vascularization are therefore achieved.

By way of an appropriate wavelength and of a high emission power, the scalp 3 absorbs in an optimum manner the mixture of step B, optimizing its photodynamic action.

The use in sequence of the three steps of the method leads to a result which is not only the sum of the advantages of each individual step but is the blending of the individual actions, with consequent extreme completeness and effectiveness.

The massage with suction of step A of the scalp 3 stimulates surface blood circulation, leading to better oxygenation of the tissue. Once the mixture has penetrated the hair follicle, it is metabolized (step B) and therefore stimulates the ribosomes to perform protein synthesis, with consequent hair growth, thus reducing to a minimum the depletion of germinal cells.

The subsequent application of the helmet 9, the light-based treatment, starts the cell biostimulation process. The energy of the photons is absorbed by the skin and by the subcutaneous layers and stimulates cell activity and the metabolism of the cell itself.

Further, the activation of the cellular mechanisms allows to activate an optimum process for mixture absorption.

The method 1 according to the invention distinctly improves hair quality even in the state that precedes non-viability of the follicle. The system activates acceleration of cell metabolism and allows optimum treatment of the scalp 3 and of the hair bulb.

It has thus been shown that the invention achieves the proposed aim and objects.

The invention thus conceived is susceptible of numerous modifications and variations, all of which are within the scope of the appended claims.

All the details may further be replaced with other technically equivalent ones.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A cosmetic method for treating the scalp (3) to slow and prevent hair loss, which consists in:
- performing, during a first step (A), a mechanical massage with suction of at least one portion (4) of the surface of the scalp (3);
- arranging, during a second step (B), on the at least one portion (4) of the surface of the scalp (3), a mixture which comprises, in proportions which can vary from 0,01% to 30%, a chemical compound for use as a cosmetic product having the formula C₉H₁₅N₅O;
- subjecting, during a step (C), the at least one portion (4) of the surface of the scalp (3) to the luminous radiation emitted by a plurality of light-emitting diodes (5); the mechanical massage with suction performed during said first step (A) consisting in arranging on the portion (4) of the surface to be treated of the scalp (3) a suction inlet (6), which is adapted to determine a partial vacuum on the skin that faces said inlet (6) and is delimited thereby; said suction inlet (6) comprising massaging means (7) which subject to a mechanical treatment the portion (4) of surface to be treated of the scalp (3) that faces said inlet (6) and is proximate thereto; said suction inlet (6) being movable by an operator along the surface of the scalp (3); said diodes (5) being suitable to subject the scalp (3) of a patient (2), wearing a helmet (9) provided on its internal surface (17) with said diodes (5), to luminous biostimulation at a wavelength and emission power adapted to enable enhanced absorption of the mixture in the scalp (3); said biostimulating radiation comprising at least one among the following light beams: a red light beam to stimulate collagen production, a blue light beam to contrast bacterial actions, a yellow light beam to stimulate the lymphatic system and the nervous system; said diodes (5) being all arranged at the same distance from the portion (4) of the surface to be treated, said distance being calculated as a function of the characteristics of the intensity and frequency of the beam emitted by the particular type of diode (5).

2. The method according to claim 1, **characterized in that** said mixture is distributed on the portion (4) of the surface to be treated of the scalp (3) equally in a localized manner and in a uniform manner.

3. The method according to claim 1, **characterized in that** said exposure to luminous radiation emitted by light-emitting diodes (5), LEDs, comprises use of radiation suitable to provide biostimulation of the photoreceptors and of the deeper layers of the skin and subcutaneous layer for better oxygenation and vascularization.

4. A kit for performing the cosmetic method of claim 1, comprising a mixture comprising, in proportions which can vary from 0.01 % to 30%, a chemical compound for use as a cosmetic product having the formula C₉H₁₅N₅O, a suction apparatus (8) and a photostimulation helmet (9), said helmet (9) being provided, on its internal surface (17), with a plurality of light-emitting diodes (5), said diodes (7) provided with the helmet (9) being distributed on multiple rows, with the purpose of affecting simultaneously with a light beam the entire scalp (3); said suction apparatus (8) being provided with at least one end inlet (6) adapted to be placed adjacent to a portion (4) of the surface to be treated of the scalp (3) and said suction producing a massage of said portion (4), said suction apparatus (8) comprising at least one suction pump (10), at least one duct (11) which is connected to at least one handpiece (12) which is provided with said at least one suction inlet (6); said handpiece (12) comprising suitable movable elements (7), which face outward proximate to said at least one suction inlet (6) for a mechanical massaging of the portion (4) of the surface to be treated; said diodes (5) being suitable to subject the scalp (3) of a patient (2), wearing said helmet (9) provided on its internal surface (17) with said diodes (5), to luminous biostimulation; said diodes (5) being adapted to emit a red light beam to stimulate collagen production, or to emit a blue light beam to contrast bacterial actions, or to emit a yellow light beam to stimulate the lymphatic system and the nervous system.

5. The kit according to the preceding claim, **characterized in that** said movable elements (7) are the massaging members (7) and are connected to a respective power supply circuit (15).

## Patentansprüche

1. Kosmetisches Verfahren zum Behandeln der Kopfhaut (3), um Haarverlust zu verlangsamen und zu verhindern, umfassend:
Ausführen einer mechanischen Massage mit Saugwirkung an mindestens einem Abschnitt (4) der Oberfläche der Kopfhaut (3) während eines ersten Schrittes (A),
Anordnen einer Mischung, die in variierenden Anteilen von 0,01 bis 30 % eine chemische Verbindung der Formel C9H15N50 zur Verwendung als kosmetisches Produkt umfasst,
auf dem mindestens einen Abschnitt (4) der Oberfläche der Kopfhaut (3) während eines zweiten Schrittes (B),
Behandeln des mindestens einen Abschnitts (4) der Oberfläche der Kopfhaut (3) mit Lichtstrahlung, die von mehreren Leuchtdioden (5) emittiert wird, während eines Schrittes (C),
wobei die mechanische Massage mit Saugwirkung, die während des ersten Schrittes (A) ausgeführt wird, im Anordnen eines Saugeinlasses (6) auf dem Abschnitt (4) der zu behandelnden Oberfläche der Kopfhaut (3) besteht, wobei der Saugeinlass (6) dafür eingerichtet ist, einen teilweisen Unterdruck auf der Haut zu bestimmen, die ihm gegenüberliegt und die ihn dadurch begrenzt, wobei der Saugeinlass (6) Massagemittel (7) umfasst, die den Abschnitt (4) der zu behandelnden Oberfläche der Kopfhaut (3), die dem Einlass (6) gegenüber- und nahe diesem liegt, einer mechanischen Behandlung unterziehen, wobei der Saugeinlass (6) durch einen Bediener über die Oberfläche der Kopfhaut (3) bewegt werden kann, wobei die Dioden (5) dafür geeignet sind, die Kopfhaut (3) eines Patienten (2), der einen Helm (9) trägt, der an seiner Innenfläche (17) mit den Dioden (5) ausgestattet ist, einer biologischen Stimulation mit Licht mit einer Wellenlänge und Emissionsleistung zu unterziehen, die dafür eingerichtet ist, eine verstärkte Absorption der Mischung in die Kopfhaut (3) zu ermöglichen, wobei die biologisch stimulierende Bestrahlung mindestens einen der folgenden Lichtstrahlen umfasst: Rotlicht, um die Collagenproduktion zu stimulieren, blaues Licht, um bakteriellen Wirkungen entgegenzutreten, gelbes Licht, um das Lymph- und das Nervensystem zu stimulieren, wobei die Dioden (5) alle in gleichem Abstand zum Abschnitt (4) der zu behandelnden Oberfläche liegen, wobei der Abstand als eine Funktion der Merkmale der Intensität und Frequenz der von der speziellen Art der Diode (5) emittierten Strahlen berechnet wird.

2. Verfahren nach Anspruch 1, **dadurch gekennzeichnet, dass** die Mischung auf dem Abschnitt (4) der zu behandelnden Oberfläche der Kopfhaut (3) gleichmäßig in lokalisierter und einheitlicher Weise verteilt wird.

3. Verfahren nach Anspruch 1, **dadurch gekennzeichnet, dass** die Behandlung mit Lichtstrahlung, die von Leuchtdioden (5) ausgestrahlt wird, die Verwendung einer Strahlung umfasst, die dafür geeignet ist, eine biologische Stimulation der Fotorezeptoren und der tieferen Hautschichten sowie der subkutanen Schicht für eine bessere Sauerstoffversorgung und Vaskularisierung bereitzustellen.

4. Set zum Ausführen des kosmetischen Verfahrens nach Anspruch 1, umfassend eine Mischung, die in variierenden Anteilen von 0,01 bis 30 % eine chemische Verbindung der Formel C9H15N50 zur Verwendung als kosmetisches Produkt umfasst, eine Saugvorrichtung (8) und einen Fotostimulationshelm (9), wobei der Helm (9) an seiner Innenfläche (17) mit mehreren Leuchtdioden (5) ausgestattet ist, wobei die im Helm (9) bereitgestellten Dioden (7) in mehreren Reihen verteilt sind, mit dem Zweck, gleichzeitig mit dem Lichtstrahl die gesamte Kopfhaut (3) zu beeinflussen, wobei die Saugvorrichtung (8) mit mindestens einem Endeinlass (6) ausgestattet ist, der dafür eingerichtet ist, angrenzend an einen Abschnitt (4) der zu behandelnden Oberfläche der Kopfhaut (3) angeordnet zu werden, und die Saugwirkung eine Massage des Abschnitts (4) bewirkt, wobei die Saugvorrichtung (8) mindestens eine Saugpumpe (10) umfasst, mindestens eine Leitung (11), die an mindestens ein Handstück (12) angeschlossen ist, das mit dem mindestens einen Saugeinlass (6) ausgestattet ist, wobei das Handstück (12) geeignete bewegliche Elemente (7) für eine mechanische Massage des Abschnittes (4) der zu behandelnden Oberfläche umfasst, die nahe dem mindestens einen Saugeinlass (6) nach außen weisen, wobei die Dioden (5) dafür geeignet sind, die Kopfhaut (3) eines Patienten (2), der den Helm (9) trägt, der auf seiner Innenfläche (17) mit den Dioden (5) ausgestattet ist, einer biologischen Stimulation mit Licht zu unterziehen, wobei die Dioden (5) dafür eingerichtet sind, einen Strahl Rotlicht zu emittieren, um die Collagenproduktion zu stimulieren, oder einen Strahl blauen Lichts zu emittieren, um bakteriellen Wirkungen entgegenzutreten, oder einen Strahl gelben Lichts zu emittieren, um das Lymph- und das Nervensystem zu stimulieren.

5. Set nach dem vorhergehenden Anspruch, **dadurch gekennzeichnet, dass** die beweglichen Elemente (7) die Massageelemente (7) sind und an einen entsprechenden Stromversorgungskreis (15) angeschlossen sind.

## Revendications

1. Procédé cosmétique pour traiter le cuir chevelu (3) de manière à ralentir et éviter l'alopécie, qui consiste en :
- la mise en oeuvre, durant une première étape (A), d'un massage mécanique avec la succion d'au moins une portion (4) de la surface du cuir chevelu (3) ;
- l'agencement, durant une deuxième étape (B), sur ladite au moins une portion (4) de la surface du cuir chevelu (3), d'un mélange qui comprend, dans des proportions qui peuvent varier de 0,01 % à 30 %, un composé chimique à utiliser comme produit cosmétique répondant à la formule C9H15N50 ;
- la soumission, durant une étape (C), de ladite au moins une portion (4) de la surface du cuir chevelu (3) à la radiation lumineuse émise par une pluralité de diodes électroluminescentes (5) ; le massage mécanique avec une succion mise en oeuvre durant ladite première étape (A) consistant à agencer, sur la portion (4) de la surface à traiter du cuir chevelu (3), une entrée de succion (6) qui est adaptée pour appliquer un vide partiel sur la peau qui fait face à ladite entrée (6) et est ainsi délimitée ; ladite entrée de succion (6) comprenant un moyen de massage (7) qui soumet à un traitement mécanique la portion (4) de surface à traiter du cuir chevelu (3) qui fait face à ladite entrée (6) et est proche de celle-ci ; ladite entrée de succion (6) étant déplaçable par un opérateur le long de la surface du cuir chevelu (3) ; lesdites diodes (5) étant adaptées pour soumettre le cuir chevelu (3) d'un patient (2), qui porte un casque (9) pourvu sur sa surface interne (17) desdites diodes (5), à une biostimulation lumineuse à une longueur d'onde et avec une puissance d'émission adaptées pour permettre une absorption accrue du mélange par le cuir chevelu (3) ; ladite radiation de biostimulation comprenant au moins un faisceau parmi les faisceaux lumineux suivants : un faisceau lumineux rouge pour stimuler la production de collagène, un faisceau lumineux bleu pour contrarier les actions bactériennes, un faisceau lumineux jaune pour stimuler le système lymphatique et le système nerveux ; lesdites diodes (5) étant toutes agencées à la même distance de la portion (4) de la surface à traiter, ladite distance étant calculée comme une fonction des caractéristiques d'intensité et de fréquence du faisceau émis par le type de diode particulier (5).

2. Procédé selon la revendication 1, **caractérisé en ce que** ledit mélange est distribué sur la portion (4) de la surface à traiter du cuir chevelu (3) en parts égales de manière localisée et uniforme.

3. Procédé selon la revendication 1, caractérisé en que ladite exposition à une radiation lumineuse émise par des diodes électroluminescentes LED (5) comprend l'utilisation d'une radiation appropriée pour pourvoir une biostimulation des photorécepteurs et des couches plus profondes de la peau et d'une couche sous-cutanée pour une meilleure oxygénation et une meilleure vascularisation.

4. Kit pour mettre en oeuvre le procédé cosmétique selon la revendication 1, comprenant un mélange comportant, dans des proportions qui peuvent varier de 0,01 % à 30 %, un composé chimique à utiliser comme produit cosmétique répondant à la formule C9H15N50, un appareil de succion (8) et un casque de photostimulation (9), ledit casque (9) étant pourvu sur sa surface interne (17) d'une pluralité de diodes électroluminescentes (5), lesdites diodes (7) pourvues sur le casque (9) étant distribuées sur des rangées multiples, dans le but d'affecter simultanément avec un faisceau lumineux l'entièreté du cuir chevelu (3) ; ledit appareil de succion (8) étant pourvu d'au moins une entrée d'extrémité (6) adaptée pour être placée de manière adjacente à une portion (4) de la surface à traiter du cuir chevelu (3) et ladite succion produisant un massage de ladite portion (4), ledit appareil de succion (8) comprenant au moins une pompe de succion (10), au moins un conduit (11) qui est connecté à au moins une pièce à main (12) pourvue de ladite au moins une entrée de succion (6) ; ladite pièce à main (12) comprenant des éléments déplaçables adaptés (7), qui font face à l'extérieur près de ladite au moins une entrée de succion (6) pour un massage mécanique de la portion (4) de la surface à traiter ; lesdites diodes (5) étant adaptées pour soumettre le cuir chevelu (3) d'un patient (2), qui porte ledit casque (9) pourvu sur sa surface interne (17) desdites diodes (5), à une biostimulation lumineuse ; lesdites diodes (5) étant adaptées pour émettre un faisceau lumineux rouge pour stimuler une production de collagène, ou pour émettre un faisceau lumineux bleu pour contrarier les actions bactériennes, ou pour émettre un faisceau lumineux jaune pour stimuler le système lymphatique et le système nerveux.

5. Kit selon la revendication précédente, **caractérisé en ce que** lesdits éléments déplaçables (7) sont les éléments de massage (7) et sont connectés à un circuit d'alimentation électrique respectif (15).
